# EUROPEAN PATENT APPLICATION

(11) **EP 2 080 482 A1**
(43) Date of publication of application: **22.07.2009**
(21) Application number: 06822692.7
(22) Date of filing: 31.10.2006
(51) Int. Cl.: A61B 18/12

(54) **HIGH FREQUENCY CAUTERY ELECTRIC POWER SOURCE DEVICE**

(71) Applicant: Olympus Medical Systems Corp., Tokyo 151-0072 (JP)
(72) Inventor: MIHORI, Takashi, Tokyo 192-8512 (JP); HAYASHIDA, Tsuyoshi, Tokyo 192-8512 (JP); NAGASE, Toru, Tokyo 192-8512 (JP); ARIURA, Aya, Tokyo 192-8512 (JP)
(74) Representative: von Hellfeld, Axel
(86) International application number: PCT/JP2006/321763
(87) International publication number: WO 2008/053532

(57) **Abstract**

A high-frequency cauterization power supply apparatus is provided with an electrosurgical scalpel device. When an operator starts to excise a living tissue in a conductive liquid, high-frequency power is applied to a treatment electrode. When an output impedance detected at that time is lower than a preset value, a variable DC power source circuit (HVPS) is set to a constant-current control mode. High-frequency power including an instantaneous high voltage is intermittently applied to the treatment electrode in a state that current output is kept at a fixed value, whereby a discharging operation starts. When the output impedance value is larger than a preset value or is large, the variable DC power source circuit is set to a constant-voltage control mode, electric power having a predetermined voltage value is supplied to the treatment electrode, whereby a living tissue to be cauterized is cauterized by using an appropriate high-frequency current.

## Description

### Technical Field

The present invention relates to a high-frequency cauterization power supply apparatus for supplying high-frequency power to an apparatus used for an excision process on a living tissue.

### Background Art

Generally, in a surgical procedure involving the living tissue, transurethral resection of the prostate and transurethral resection of a bladder tumor are known as a form of manipulative surgical operation relatively low in invasiveness and directed at, for example, a patient with prostate or bladder disease. In the description to be given hereunder, these extirpation techniques will be referred simply to as "TUR". In a procedure, a surgeon supplies high-frequency power to a treatment electrode having been led to an affected part of a patient, and applies a removal procedure, such as cauterization, incision, peeling, or hemostasis, to the patient by utilizing the heating generated by the high-frequency power, while observing the affected part by the hard endoscope inserted into the urethra. In the description, including embodiment description, to be given hereunder, these removal procedures will be referred to generally as "cauterization".

Formerly, the high-frequency power source mainly used for the TUR is of the so-called mono-polar type which contains a circuitry including a treatment electrode and a counter electrode plate. In the high-frequency power of the mono-polar, the counter electrode is laid under a patient's body. Accordingly, a physical distance between an operating site and a return current becomes long. A spark discharge, which occurs when the high-frequency current flows, causes nerve reflection and involuntary movement in the patient during the manipulative surgical operation. This movement sometimes hinders the surgical operation. During the surgical operation, a nonelectrolyte solution as an under current is fed to the urethra. This sometimes brings about increase of the cost per case and produces harmful side effects such as blood hyponatremia caused by bleeding in the surgical operation. In the situation where it is required to overcome the disadvantages of the conventional TUR, attention is recently paid to a new TUR carried out in a conductive liquid. The new TUR and the conventional one are almost the same in the basic manipulative surgical technique and necessary equipment units, with some exceptions. The exceptions are that the new TUR needs no counter electrode and locates the return electrode (for example, sheath) in the vicinity of the electrode of the surgical instrument, and to use normal saline solution as an undercurrent during the surgical operation.

### Disclosure of Invention

Accordingly, an object of the present invention is to provide a high-frequency cauterization power supply apparatus which is free from cost increase, up-sizing, and excessive increase of a scale of a high-frequency power circuit, and has improved discharge startability in an environment where the conductive liquid is used.

The present invention provides a high-frequency cauterization power supply apparatus comprising: an impedance detecting unit used for a surgical instrument having a treatment electrode, which discharges in a conductive liquid upon application of high-frequency power and treats a living tissue, the impedance detecting unit detecting an impedance between the treatment electrode and the living tissue from the high-frequency power output to the treatment electrode; and a high-frequency power generator unit which generates, when the high-frequency power is applied to the treatment electrode being in a non-discharging state, first high-frequency power being intermittently output at a time period determined based on the impedance value, when an impedance value detected by the impedance detecting unit is below a preset value determined based on the living tissue, and generates a second high-frequency power being continuously output when the impedance value detected exceeds the preset value, the first and second high-frequency power being output to the treatment electrode through the impedance detecting unit.

Further, provided is a method for applying high-frequency power generated by a high-frequency cauterization power supply apparatus, the method characterized by comprising the steps of: detecting an impedance between a treatment electrode and a living tissue from the high-frequency power applied to the treatment electrode being in a non-discharging state, when high-frequency power is applied to a surgical instrument having the treatment electrode, which discharges in a conductive liquid upon application of high-frequency power and treats a living tissue; outputting intermittently first high-frequency power at a time period determined based on the impedance value, when an impedance value detected by the impedance detecting step is below a preset value determined based on the living tissue; and outputting continuously second high-frequency power when the impedance value detected exceeds the preset value.

### Brief Description of Drawings

FIG. 1 is a diagram showing a high-frequency cauterization power supply apparatus according to a first embodiment of the invention;
FIG. 2A is a waveform diagram showing a control mode signal waveform when the power supply apparatus of the first embodiment is in a constant-voltage control mode;
FIG. 2B is a waveform diagram showing a control mode signal waveform when the power supply apparatus of the first embodiment is in a constant-current control mode;
FIG. 2C is a waveform diagram showing a high-frequency power waveform when the power supply apparatus of the embodiment is in a constant-voltage control mode;
FIG. 2D is a waveform diagram showing a high-frequency power waveform when the power supply apparatus of the first embodiment is in a constant-current control mode;
FIG. 2E is a waveform diagram showing a waveform of a waveform generating signal for driving a switching circuit of the first embodiment;
FIG. 3 is a diagram for explaining a set value to switch the constant-voltage control mode and the constant-current control mode in the power supply apparatus of the first embodiment;
FIG. 4A is a diagram showing a state of a treatment electrode in a conductive liquid when high-frequency power generated according to the first embodiment is not applied thereto;
FIG. 4B is a diagram showing a state of the treatment electrode in a conductive liquid in an initial stage when high-frequency power generated according to the first embodiment is applied thereto;
FIG. 4C is a diagram showing a state of the treatment electrode in a conductive liquid when high-frequency power generated according to the first embodiment is applied thereto;
FIG. 4D is a diagram showing a state of the treatment electrode in a conductive liquid when high-frequency power generated according to the first embodiment is applied thereto and the treatment electrode is discharging;
FIG. 5 is a diagram showing a high-frequency cauterization power supply apparatus according to a modification of the first embodiment;
FIG. 6A is a diagram showing a waveform of a control mode signal in a high-frequency cauterization power supply apparatus according to a second embodiment of the invention;
FIG. 6B is a diagram showing a waveform of high-frequency power in the high-frequency cauterization power supply apparatus according to the second embodiment of the invention; and
FIG. 7 is a diagram showing a waveform of high-frequency power in the high-frequency cauterization power supply apparatus, which is a modification of the second embodiment of the invention.

### Best Mode for Carrying Out the Invention

The preferred embodiments of the present invention will be described in detail with reference to the accompanying drawings.

The present invention relates to a high-frequency cauterization power supply apparatus for supplying high-frequency power to a surgical instrument of an electrosurgical scalpel device for excising a living tissue in a conductive liquid by using high-frequency power.

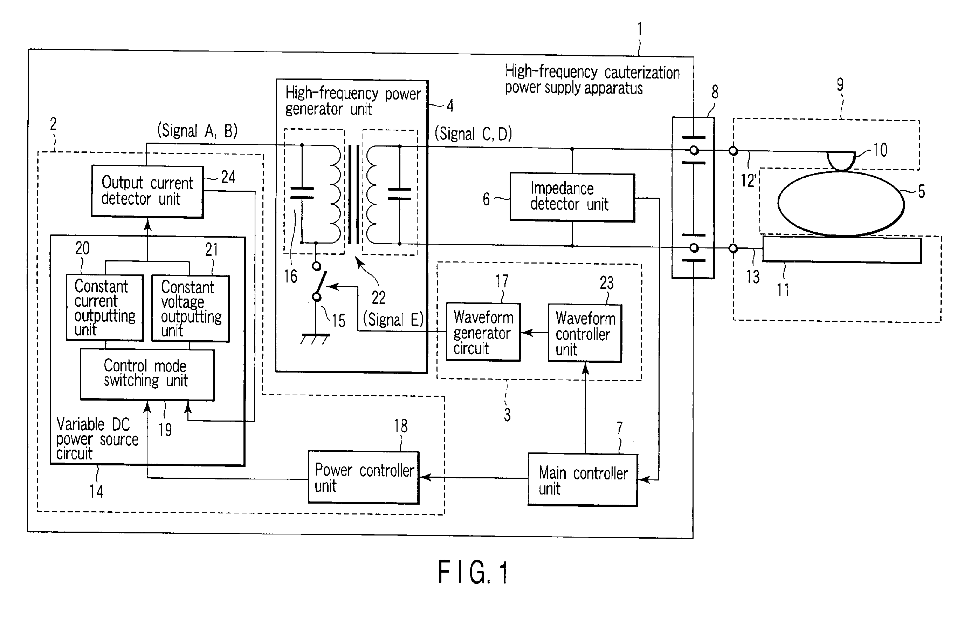

FIG. 1 is a diagram showing a configuration of a high-frequency cauterization power supply apparatus (referred to as a power supply apparatus) according to a first embodiment of the invention.

The power supply apparatus 1 is made up of a control mode signal generator unit 2 for generating and outputting a control mode signal to be described later, a waveform instructing unit 3 for designating a signal waveform of high-frequency power to be output, a high-frequency power generator unit 4 for generating a control mode signal and high-frequency power based on a signal waveform designated, an impedance detector unit 6 for detecting an impedance value from the high-frequency power output, and a main controller unit 7 for controlling all the related units in the power supply apparatus.

The power supply apparatus 1 supplies high-frequency power to a surgical instrument 9, coupled to an output terminal 8, for a required surgical procedure for a living tissue 5. The surgical instrument 9 includes a treatment electrode 10 and a return electrode 11 (uses, for example, a rigid scope sheath). High-frequency power is supplied through a lead 12 to the treatment electrode 10, and through the living tissue 5 to the return electrode 11, and finally returned to the power supply apparatus 1 via a lead 13.

The impedance detector unit 6 detects an electrical impedance, which varies according to a discharging state of the treatment electrode 10 and a variation (cauterization state) of a living tissue (referred to as a living tissue) 5 to be treated. The result of the detection is transmitted to the main controller unit 7.

The main controller unit 7, containing a preset value, compares an impedance value from the impedance detector unit 6 with the preset value. If the received impedance value is smaller than the preset value, the main controller unit issues an instruction to intermittently output high-frequency power at periods, each being determined based on the impedance value. If the received impedance value is larger than the preset value, the main controller unit issues an instruction to continuously output high-frequency power.

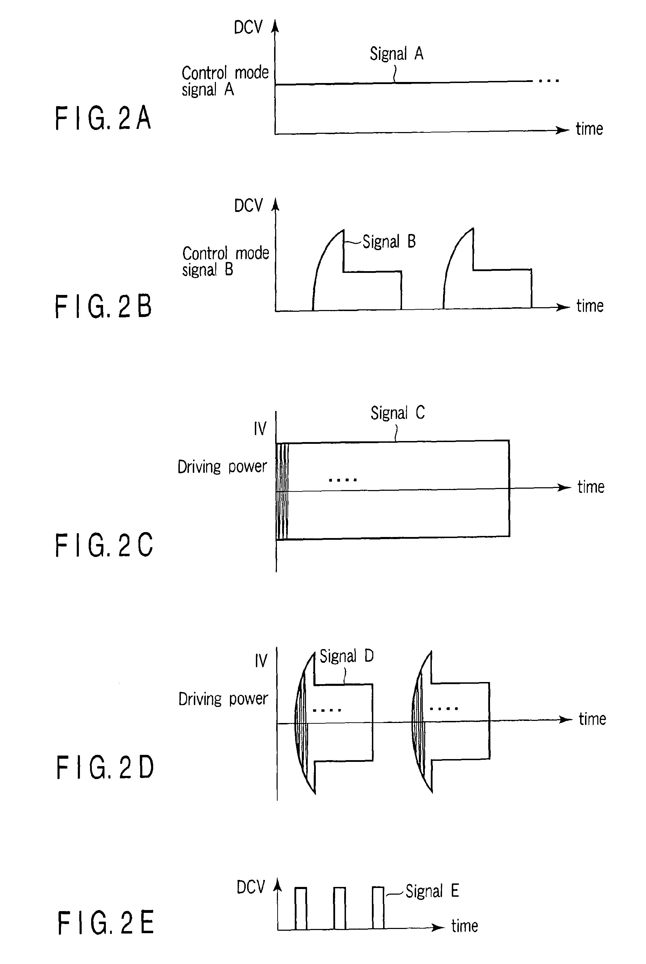

The control mode signal generator unit 2 selects one of two control modes previously set up (a constant-voltage control mode or a constant-current control mode) in accordance with a detect signal (instruction by the main controller unit 7) corresponding to an impedance value output from the impedance detector unit 6, and outputs a control mode signal representing either of the control modes to the high-frequency power generator unit 4. The control mode signal indicates DC power generated by the control mode signal generator unit 2 (FIGS. 2A and 2B). A voltage value in the constant-voltage control mode and a current in the constant-current control mode may be appropriately set within a range of the rated output. The control mode signal generator unit 2 includes a variable DC power source circuit (HVPS) 14 for selecting the constant-voltage control mode or the constant-current control mode and outputting a control mode signal in the selected control mode, a power controller unit 18 for controlling the variable DC power source circuit (HVPS) 14 in accordance with an instruction by the main controller unit 7, and an output current detector unit 24 for detecting a current component from the control mode signal A or B output from the variable DC power source circuit 14.

The variable DC power source circuit 14 includes a control mode switching unit 19 for selecting the constant-voltage control mode or the constant-current control mode in accordance with a control signal from the power controller unit 18, a constant voltage outputting unit 21 for outputting a control mode signal A shown in FIG. 2A in accordance with the control mode selected when the power supply apparatus is in the constant-voltage control mode, and a constant current outputting unit 20 for outputting a control mode signal B shown in FIG. 2B in accordance with the control mode selected. The constant voltage outputting unit 21 and the constant current outputting unit 20 are separately explained for ease of explanation. Actually, those units may be designed as one circuit. In this case, the circuit outputs one of different output signals by control.

In the present embodiment, a control mode signal A shown in FIG. 2A is output in the constant-voltage control mode, and a control mode signal B shown in FIG. 2B is output in the constant-current control mode. In the control mode switching unit 19, the output impedance value for switching the control mode is set at about 50Ω in this instance. When a control signal indicative of 50Ω as the impedance value (a detect signal from the power controller unit 18 or a detect signal from the output current detector unit 24) is received, the control mode is switched between the constant-voltage control mode and the constant-current control mode. The output impedance value used for the mode switching is not limited to 50Ω, as a matter of course. It may be set according to a kind and a state of the living tissue to be medically treated.

The output current detector unit 24 detects the control mode signal A or the control mode signal B output, and generates a detect signal for the feedback control for the mode switching on the basis of the detected current component to be output to the control mode switching unit 19.

The high-frequency power generator unit 4 is made up of a high-frequency output transformer 22 including primary and second coils, electromagnetic-induction coupled, which have appropriate turn ratios, a parallel resonance circuit 16 coupled to the primary coil of the high-frequency output transformer 22, and a switching circuit 15, coupled likewise to the primary coil, for generating a high-frequency signal at a desired frequency by swinging the control mode signal A or B as a DC voltage signal input at an amplitude. The parallel resonance circuit 16 of the primary side has main functions; a function to remove a spurious component from a rectangular wave signal resulting from the switching operation, and another function to boost the signal.

The waveform instructing unit 3 is made up of a waveform generator circuit 17 for generating a switching signal E shown in FIG. 2E to drive the switching circuit 15, and a waveform controller unit 23 for controlling the waveform generator circuit 17. The waveform controller unit 23 receives a control signal for generating a waveform from the main controller unit 7. The control signal is generated in accordance with each output mode that is set on a setting panel, not shown, and an output set value, and on the basis of a signal corresponding to an impedance value detected by the impedance detector unit 6.

The power supply apparatus of the embodiment thus far described has the following features.

A first feature is to detect an output impedance value of the high-frequency cauterization power supply apparatus, which indicates a discharging state (and a cauterization state of the living tissue) of the treatment electrode. A second feature is that the variable DC power source circuit (HVPS) in the power supply apparatus has two control modes, the constant-voltage control mode and the constant-current control mode. A third feature is that when the impedance value is lower than a preset value, the high-frequency power is intermittently output at periods each determined according to an impedance value at that time, and when the impedance value is higher than a preset value, the high-frequency power is continuously output. A fourth feature is that the switching unit is used which selects the control mode according to the impedance value: it selects the constant-voltage control mode when the impedance value is higher than the preset value, and the constant-current control mode when the former is lower than the latter. The impedance values of the third and the fourth features are not always equal to each other, but may be selected independently. In the embodiment, the transformer and the switching circuit are combined for generating the high-frequency power. It is evident that another electronic approach is present in addition of the combination. It is a combination of a coil and a capacitor or a combination of a frequency adjustable oscillation circuit using a crystal oscillation element and an amplifier circuit using transistors and others.

Next, description is given about the constant-voltage control mode and the constant-current control mode of the power supply apparatus of the embodiment. A discharging operation in a conductive solution is first described.

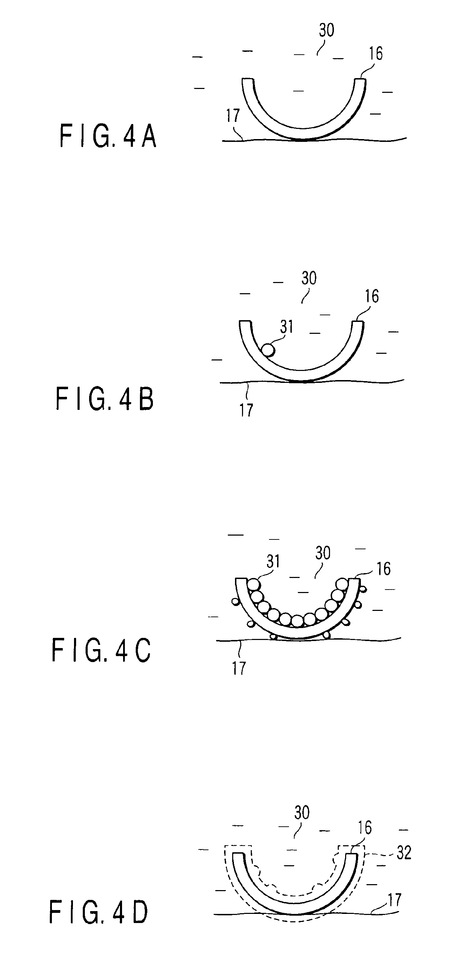

The power supply apparatus drives an electrosurgical scalpel device used for performing surgical procedures, such as excision, coagulation, and vaporization of a living tissue in a conductive solution. As shown in FIG. 4A, no bubble is attached to the treatment electrode 10 not applied with high-frequency power in a conductive solution 30. As shown in FIG. 4B, when the high-frequency power is applied to the electrode, it warms normal saline solution in the vicinity of the treatment electrode 10 by the energy of the high-frequency power to generate bubbles. As shown in FIG. 4C, when the application of the high-frequency power and the feeding of the energy to the treatment electrode 10 are further continued, an amount of bubbles increases to entirely cover the electrode. At this time point, an impedance value (or called an output impedance value) among the electrode, the normal saline solution and the living tissue abruptly increases, and high voltage is applied to the treatment electrode. Then, as shown in FIG. 4D, discharging starts. Thus, to start the discharging operation, the effective way is to instantaneously apply high power (voltage). By so doing, the bubbles are quickly generated to thereby increase the impedance value.

The normal electrosurgical scalpel device sometimes exhibits about 100Ω or higher as the impedance value. Within such an impedance range, the normal variable DC power source circuit 14 will be controlled so as to produce a fixed voltage irrespective of the output impedance value, in the constant-voltage control mode. In the TUR in the conductive liquid, if the constant-voltage control is performed in a state that the initial impedance value is as low as 30Ω, the high-frequency power (current value) output from the power supply apparatus is extremely high. Necessity arises to deal with the increase of the current value, however. The necessity needs an overall design of the power supply apparatus, allowing for the heat radiation and derating in the power supply apparatus. The result is to increase the size of the power supply apparatus. Use of high voltage withstand units can cope with the high voltage problem. In this respect, this problem does not directly lead to the up-sizing of the power supply apparatus. As described above, to enhance the startability in the conductive liquid, high power or voltage is essential.

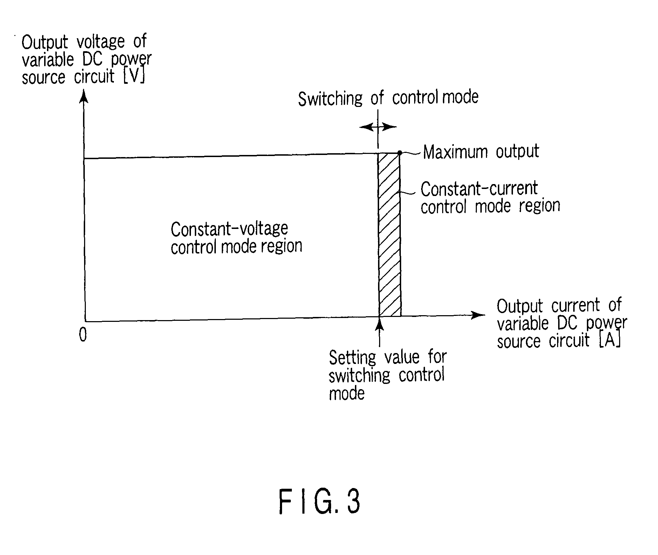

In the embodiment, at the start of the high-frequency power application, if it is detected that the impedance value is below a preset value (low impedance value) to switch the control mode as shown in FIG. 3, it is controlled such that the control mode of the power supply apparatus is shifted to the constant-current control mode to increase instantaneous high-frequency power, which in turn is applied to electrode, and after the voltage application, the high-frequency power is reduced. In the embodiment, the control mode switching is performed on the basis of a detection result of an output current detecting unit 240 for detecting an output current from the variable DC power source circuit, which has a correlation with an output impedance. An instantaneous voltage that is high enough to trigger the discharging operation suffices for the high voltage and after the discharging operation starts, there is no need of continuing the high voltage state. In the constant-voltage control mode of the embodiment, as shown in FIG. 2B, one control mode signal B is configured in waveform such that its amplitude is kept at a fixed value, instantaneously and sharply rises, and then falls to be kept at a fixed value. The interval of outputting the control mode signal B is adjustable in accordance with a magnitude of the output impedance.

When the constant-current control mode is set up, the output voltage value is within the rated output, and the integral power consumption of the electric power applied to the living tissue for each unit time or a given time period is controlled so as to perform an appropriate cauterization. In the embodiment, to make the integral power consumption per unit time constant, the voltage application is stopped for a time period corresponding to the electric energy defined by the applied high voltage. With provision of the voltage application-ceasing period, it is allowed that the integral power consumption is adjusted and high voltage is applied. Thus, the high voltage is intermittently varied in accordance with a predetermined algorithm so that the discharging operation starts at the treatment electrode. This feature brings about such advantages that the high-frequency power does not exceed the rate output, and excessive high-frequency power is not applied to the living tissue to be subjected to the surgical procedure.

For the constant-voltage control mode, the control mode signal A is generated as a continuous DC voltage signal, as shown in FIG. 2A. Those control mode signals A and B are input to one end of the primary coil of the high-frequency output transformer 22 in the high-frequency power generator unit 4. At this time, signal waveforms (FIGS. 2A and 2B) of the control mode signals from the high-frequency output transformer 22 appear, and high-frequency power is output, of which a frequency is based on the driving by the switching circuit 15, which operates according to the output signal of the waveform generator circuit 17. Actually, at the start of applying the high-frequency power, the power supply circuit is started in the constant-voltage control mode in consideration of the case where the operator continuously uses the electrosurgical scalpel device. If required, the circuitry is modified such that when a low output impedance value, i.e., a low impedance of the electrosurgical scalpel device, is detected, the control mode is immediately switched to a constant-current control mode and the discharging operation based on the intermittent power application is performed.

The intermittent outputting mode of the high-frequency power will be described.

As in the embodiment mentioned above, the outputting of the high-frequency power is controlled such that an intermittent outputting mode of the high-frequency power is performed before the discharging operation starts, and after the discharging operation starts, a continuous outputting mode is performed. An impedance threshold value for switching between the intermittent outputting mode and the continuous outputting mode may be 200Ω. Examples of duty cycle of the intermittent outputting follows. When an output impedance detected by the impedance detector unit 6 is Z, Z < 80Ω : about 67% (100 ms/50 ms), 80 ≤ Z < 200Ω : 50% (50 ms/50 ms), and Z > 200Ω : 100% (continuous). If required, those settings may be appropriately changed. When the duty cycle is changed according to the impedance value, it is required that as the impedance value is smaller, the duty becomes higher. By increasing the duty, the average power per unit time can be obtained without increasing the capacity of the power supply circuit even in a low impedance region.

Parameters for judging the switching operation may be 1) time, 2) a time changing rate of an electrical impedance for each of the values of the output voltage, output current, and output power from the high-frequency power generator unit 4, and the impedance value, or 3) a phase difference between the output voltage and the output current from the high-frequency power generator unit 4. As for the phase difference, when a phase judging threshold value is 45° or less, the first high-frequency power is intermittently output. When it exceeds 45°, the second high-frequency power is continuously output. Also in a crest factor to be given later, 1) time, 2) a time changing rate of an electrical impedance for each of the values of the output voltage, output current, and output power from the high-frequency power generator unit 4, and the impedance value, or 3) a phase difference between the output voltage and the output current from the high-frequency power generator unit 4, are used.

As for the phase difference, when a phase judging threshold value is 45° or less, an output value by a first crest factor based on the first high-frequency power is output. When it exceeds 45°, an output value by a second crest factor based on the second high-frequency power is output.

As described above, when an excision process starts in the conductive liquid by using the electrosurgical scalpel device using the high-frequency cauterization power supply apparatus of the embodiment, the control mode of the variable DC power source circuit (HVPS) is switched form the constant-voltage control mode to the constant-current control mode according to an output impedance value, and a high-frequency power containing an instantaneous high voltage value is intermittently applied to the electrode. The constant voltage and the constant current are within the rated output, and the living tissue is cauterized by a proper high-frequency power. The high-frequency cauterization power supply apparatus of the embodiment can withstand high voltage. Therefore, the heat radiation design and derating are easy compared to the circuit construction outputting large current, and the power supply circuit is realized while free from up-sizing. Accordingly, without large cost increase, the treatment electrode of the electrosurgical scalpel device starts its operation for a short time and the operator can quickly start the surgical operation, when compared to the conventional power supply apparatus. Since the constant-voltage control mode is switched to the constant-current control mode on the basis of detection of the output impedance value, a living tissue to be cauterized is cauterized by a proper high-frequency power.

A modification of the first embodiment described above will be described.

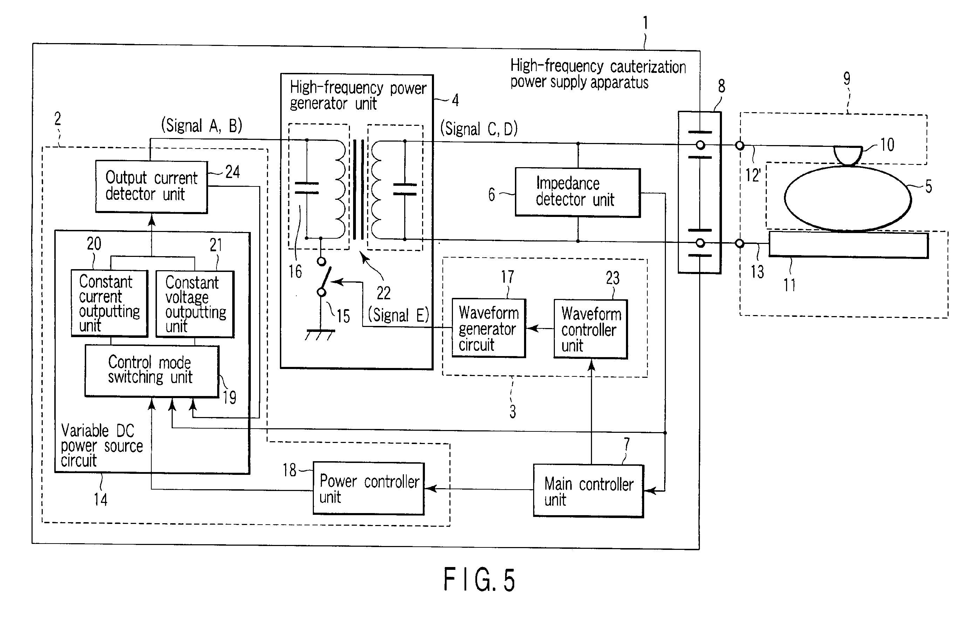

FIG. 5 is a diagram showing a power supply apparatus according to a modification of the first embodiment. In the figure, like or equivalent portions are designated by like reference numerals in the first embodiment, and the detailed description thereof will be omitted.

In the modification, a signal representing the detection result from the impedance detector unit 6 connected to the output terminal of the high-frequency power generator unit 4 is input to the control mode switching unit 19.

The modification also achieves the useful effects equivalent to those by the first embodiment. In the modification, the switching of the control mode to the constant-current control mode is quicker than that in the circuit arrangement where the detection result is input to the control mode switching unit 19 by way of the main controller unit 7 and the power controller unit 18 as in the first embodiment, since the detection result is directly input to the control mode switching unit.

A second embodiment of the present invention will be described.

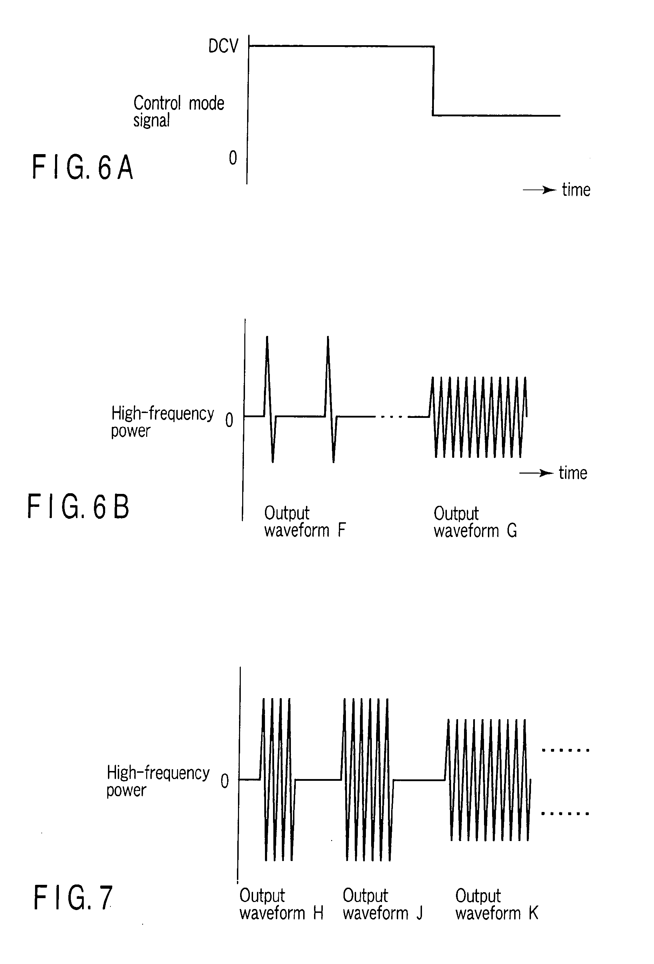

The second embodiment is a high-frequency cauterization power supply apparatus which generates high-frequency power based on a control mode signal having a peak value (voltage), which is based on an output impedance value, and applies the high-frequency power to the electrosurgical scalpel device. In the embodiment, like or equivalent portions are designated by like reference numerals in the first embodiment shown in FIG. 1 and the detailed description thereof will be omitted. FIG. 6A is a diagram showing a waveform of a control mode signal (output voltage 1 of the variable DC power source circuit 14), and FIG. 6B shows a waveform of high-frequency power.

The second embodiment is substantially the same as the first embodiment in that the high-frequency power voltage is instantaneously increased till the discharging operation starts. A technical means to instantaneously increase the power voltage of the present embodiment is different from that of the first embodiment.

In the embodiment, as shown in FIG. 6B, high-frequency power having a waveform F with a relatively high crest factor is output till the discharging operation starts, and when it starts, the high-frequency power having a waveform G with a low crest factor is output.

For the control mode signal, parameters for judging the switching operation may be 1) time, 2) a time changing rate of electrical parameters in each of the values of the output voltage, output current, output power, and the impedance value, 3) a discharging state (realized by detecting a low frequency component due to rectifying effect or detecting harmonic at the time of discharge), and 4) a phase difference between the output voltage and the output current. These parameters may be individually used or a plurality of them may be combined.

The term "crest factor" means the quotient of dividing a peak value (high-frequency waveform) per unit time by an effective value (DC energy converted from the energy of an AC waveform). When two waveforms having equal output power per unit time are compared, a voltage value (crest value) whose waveform has a high crest factor is high. In other words, it means that the applied voltage that instantaneously rises is high.

The second embodiment produces useful effects comparable with those of the first embodiment by applying such an output voltage of high crest factor to the electrode.

Further, the instantaneous voltage (power), which is higher than that of the first embodiment, is applied to the related electrode, so that the discharging starts faster than in the first embodiment. Generally, the electrosurgical scalpel device is designed so as to produce an excision waveform (crest factor: low) for excising the tissue, and a coagulation waveform for coagulating the tissue (crest factor: high).

In the present embodiment, if a waveform switching circuit is additionally used, there is no need of using additional components. The startability of the power supply apparatus is enhanced while being free from size increase, cost increase and reliability deterioration. In the embodiment, the voltage waveform of high crest factor is generated through the peak value control by the control mode signal A from the constant voltage outputting unit 21 and the driving of the switching circuit 15 by the waveform generator circuit 17.

It is known that the voltage waveform of high crest factor provides a high coagulation of the tissue. Therefore, by applying the coagulation waveform to the treatment electrode at the initial stage of the power application, the conductive liquid is heated, and further a tissue to be a cauterized may be cauterized in advance, providing hemostatic effect.

As described above, according to the present embodiment, when the waveform of high crest factor is applied to the electrode till the discharging operation starts, instantaneous energy is increased to improve the startability of the discharging operation.
Further, an excision mode and a coagulation mode, which are contained in the electrosurgical scalpel device, are switched from one to the other and vice versa. This eliminates the necessity of increasing the current capacity of the circuit in the power supply system in order to improve the startability of the discharging operation.

A modification of the second embodiment is described.

FIG. 7 is a diagram showing a waveform of high-frequency power output from the power supply apparatus. In the second embodiment, when the discharging operation starts, the high-frequency power applied to treatment electrode takes a one amplitude waveform or high-frequency power having a waveform resembling that waveform. In the modification, high-frequency power having output waveforms H and J, which are continuous for time widths (number of amplitudes) is applied to the treatment electrode. At instant that a discharging operation starts at the treatment electrode, high-frequency power having a waveform K for cauterization process is output. The time width for the continuous outputting is determined by an output voltage value (peak value) and the output impedance value, and within the rated output.

The modification produces useful effects comparable with those of the second embodiment. Additionally, the time duration of the high voltage is longer, so that the discharging operation is easier to start.

The embodiment mentioned above involves the following invention.
(1) A method of applying high-frequency power to a high-frequency cauterization power supply apparatus, the method comprising a step of detecting an impedance between a treatment electrode and a living tissue from high-frequency power applied to the treatment electrode being in a non-discharging state when the high-frequency power is applied to a surgical instrument having the treatment electrode, which discharges in a conductive liquid upon application of the high-frequency power and treats the living tissue, wherein when an impedance value detected by the impedance detecting unit is below a preset value determined based on the living tissue, first high-frequency power having a first crest factor determined based on the impedance value is output, and when the impedance value detected exceeds the preset value, second high-frequency power having a second crest factor lower than the first crest factor is output.

## Claims

1. A high-frequency cauterization power supply apparatus **characterized by** comprising:
an impedance detecting unit used for a surgical instrument having a treatment electrode, which discharges in a conductive liquid upon application of high-frequency power and treats a living tissue, the impedance detecting unit detecting an impedance between the treatment electrode and the living tissue from the high-frequency power output to the treatment electrode; and
a high-frequency power generator unit which generates, when the high-frequency power is applied to the treatment electrode being in a non-discharging state, first high-frequency power being intermittently output at a time period determined based on the impedance value, when an impedance value detected by the impedance detecting unit is below a preset value determined based on the living tissue, and generates a second high-frequency power being continuously output when the impedance value detected exceeds the preset value, the first and second high-frequency power being output to the treatment electrode through the impedance detecting unit.

2. The high-frequency cauterization power supply apparatus according to claim 1, **characterized in that** a maximum amplitude of the first high-frequency power output from the high-frequency power generator unit is higher that that of the second high-frequency power.

3. The high-frequency cauterization power supply apparatus according to claim 1, **characterized by** further comprising:
a variable DC power source circuit including
a control mode switching unit which selects a constant-current control mode signal providing a source of the first high-frequency power output from the high-frequency power generator unit or a constant-voltage control mode signal providing a source of the second high-frequency power based on the detection result of the impedance detecting unit and outputs the selected control mode signal,
a constant current output unit which outputs the constant-current control mode signal in response to an instruction of the control mode switching unit, and
a constant voltage output unit which outputs the constant-voltage control mode signal according to an instruction from the control mode switching unit; and
a switching circuit which is provided in the high-frequency power generator unit and assigns an appropriate frequency to the constant-current control mode signal and the constant-voltage control mode signal output from the variable DC power source circuit.

4. The high-frequency cauterization power supply apparatus according to claim 3, **characterized by** further comprising an output current detecting unit which detects a current component from the constant-current control mode signal and the constant-voltage control mode signal output from the variable DC power source circuit, in addition to the detect result from the impedance detecting unit, and feeds the current component back to the control mode switching unit for the switching control.

5. The high-frequency cauterization power supply apparatus according to claim 3, **characterized by** further comprising a controller unit which determines as the preset value an impedance value at which a non-discharging state transits to a discharging state in the treatment electrode applied with the high-frequency power.

6. The high-frequency cauterization power supply apparatus according to claim 1, **characterized in that** the high-frequency power generator unit includes
a high-frequency output transformer having a proper turn ratio and being electromagnetically coupled by a coil, and
a switching circuit which is coupled to a primary coil of the high-frequency output transformer and generates a high-frequency signal at a desired frequency by swinging the constant-current control mode signal or the constant-voltage control mode signal, each consisting of a DC voltage signal input.

7. The high-frequency cauterization power supply apparatus according to claim 6, **characterized by** further comprising a parallel resonance circuit, coupled to the primary coil of the high-frequency output transformer of the high-frequency power generator unit, having a function to remove a spurious component from a rectangular wave signal resulting from the switching operation of the switching circuit, and another function to boost the signal.

8. The high-frequency cauterization power supply apparatus according to claim 1, **characterized in that** the high-frequency power generator unit uses time as a judging parameter for switching between intermittent output and continuous output by switching between the first high-frequency power and the second high-frequency power.

9. The high-frequency cauterization power supply apparatus according to claim 1, **characterized in that** the high-frequency power generator unit uses a time changing rate of an electrical impedance for any of the values of the output voltage, output current, output power, and impedance value as the judging parameter for switching between intermittent output and continuous output by switching between the first high-frequency power and the second high-frequency power.

10. The high-frequency cauterization power supply apparatus according to claim 1, **characterized in that** the high-frequency power generator unit uses a phase difference of one of an output voltage and an output current as the judging parameter for switching between intermittent output and continuous output by switching between the first high-frequency power and the second high-frequency power.

11. The high-frequency cauterization power supply apparatus according to claim 10, **characterized in that** as for the phase difference in the judging parameter, when a phase judging threshold value is 45° or less, the first high-frequency power is intermittently output, and when the phase judging threshold value exceeds 45°, the second high-frequency power is continuously output.

12. A high-frequency cauterization power supply apparatus **characterized by** comprising:
an impedance detecting unit used for a surgical instrument having a treatment electrode, which discharges in a conductive liquid upon application of high-frequency power and treats a living tissue, the impedance detecting unit detecting an impedance between the treatment electrode and the living tissue from the high-frequency power output to the treatment electrode; and
a high-frequency power generator unit which generates, when the high-frequency power is applied to the treatment electrode being in a non-discharging state, first high-frequency power being output at a first crest factor determined based on the impedance value, when an impedance value detected by the impedance detecting unit is below a preset value determined based on the living tissue, and generates a second high-frequency power being output at a second crest factor lower than the first crest factor of the first high-frequency power when the impedance value detected exceeds the preset value, the first and second high-frequency power being output to the treatment electrode through the impedance detecting unit.

13. The high-frequency cauterization power supply apparatus according to claim 12, **characterized in that** the first crest factor of the high-frequency power waveform output till a discharging operation starts is at least more than 2.

14. The high-frequency cauterization power supply apparatus according to claim 12, **characterized in that** the second crest factor of the high-frequency power waveform output after the discharging operation starts is more than 1 and less than 2.

15. The high-frequency cauterization power supply apparatus according to claim 12, **characterized in that** the high-frequency power generator unit uses time as a judging parameter for switching between an output value by the first crest factor and an output value by the second crest factor by switching between the first high-frequency power and the second high-frequency power.

16. The high-frequency cauterization power supply apparatus according to claim 12, **characterized in that** the high-frequency power generator unit uses a time changing rate of an electrical impedance for any of the values of the output voltage, output current, output power, and impedance value,
as a judging parameter for switching between an output value by the first crest factor and an output value by the second crest factor by switching between the first high-frequency power and the second high-frequency power.

17. The high-frequency cauterization power supply apparatus according to claim 12, **characterized in that** the high-frequency power generator unit uses a phase difference of one of an output voltage and an output current as a judging parameter for switching between an output value by the first crest factor and an output value by the second crest factor by switching between the first high-frequency power and the second high-frequency power.

18. The high-frequency cauterization power supply apparatus according to claim 17, **characterized in that** as for the phase difference in the judging parameter, when a phase judging threshold value is 45° or less, the first high-frequency power defined by the first crest factor is output, and when the phase judging threshold value exceeds 45°, the second high-frequency power defined by the second crest factor is output.

19. A method for applying high-frequency power generated by a high-frequency cauterization power supply apparatus, the method **characterized by** comprising the steps of:
detecting an impedance between a treatment electrode and a living tissue from the high-frequency power applied to the treatment electrode being in a non-discharging state, when high-frequency power is applied to a surgical instrument having the treatment electrode, which discharges in a conductive liquid upon application of high-frequency power and treats a living tissue;
outputting intermittently first high-frequency power at a time period determined based on the impedance value, when an impedance value detected by the impedance detecting step is below a preset value determined based on the living tissue; and
outputting continuously second high-frequency power when the impedance value detected exceeds the preset value.
